# EUROPEAN PATENT SPECIFICATION

(11) **EP 1 448 142 B1**
(45) Date of publication and mention of the grant of the patent: **18.08.2010**
(21) Application number: 02802755.5
(22) Date of filing: 01.11.2002
(51) Int. Cl.: A61F 13/20

(54) **ABSORBENT PRODUCT**
ABSORBIERENDES PRODUKT
PRODUIT ABSORBANT

(30) Priority: 08.11.2001 SE 0103704
(43) Date of publication of application: 25.08.2004
(73) Proprietor: SCA Hygiene Products AB, 405 03 Göteborg (SE)
(72) Inventor: LUNDIN, Catarina, S-436 40 Askim (SE)
(74) Representative: Romare, Laila Anette
(86) International application number: PCT/SE2002/001982
(87) International publication number: WO 2003/039424

(56) References cited:
- EP-A1- 0 249 924
- FR-A- 2 400 356
- GB-A- 2 041 757
- GB-A- 2 148 125
- US-A- 3 688 771

## Description

### TECHNICAL FIELD

The invention relates to an absorbent product such as a sanitary towel, an incontinence pad, a panty liner or the like intended to be worn in a pair of briefs. This type of product is provided with a means of attachment which is intended for fastening the product in a pair of briefs. The means of attachment is covered by a release strip which is removed when the means of attachment is used.

### BACKGROUND ART

Modern sanitary towels, incontinence pads, panty liners and the like are in most cases provided with an attachment system for fastening these in a pair of briefs. The means of attachment can be in the form of, for example, adhesive, touch-and-close fasteners or snap fasteners. It is common to all types of means of attachment that they should be protected before use. One way in which it is possible to protect these is by using what is known as a release strip, that is to say a paper strip or the like which has been treated so as to be easily removable from the means of attachment when the absorbent product is used. The release paper usually projects beyond the periphery of the attachment so as to create an area under the release paper which is adhesive-free and forms a tab which facilitates removal of the release paper. A problem which can then arise with the products available today is that it can be difficult to take hold of the release paper in order to pull it away. This is a problem which many elderly people may experience because fine psychomotor skills often deteriorate with increasing age. Rheumatics and other disabled people with reduced mobility in the hands may also have the same problem. Other instances when it can be difficult to take hold of the release paper may be when the hands are cold, moist or slippery on account of, for example, skin creams or the like.

One way of solving the problem is described in EP 0 680 740 A1 which describes a product in which the release paper is divided into more than one part along a line which is preferably not parallel to a longitudinal line running in the length direction of the product. In order for it to be possible to detach the release paper, it is therefore necessary to bend the product so that the paper divides. Such a solution means that several handling stages are required in order to detach the release paper from the means of attachment. First the product has to be bent and then, when the release paper comes away, first a first part and then a second part of the release paper has to be removed and thrown away. In this procedure, it is not certain either that the release paper will actually come away from the means of attachment so that it will be possible to take hold of the release paper.

Another variant of release paper is described in EP 0 313 426 A1. This document describes a release paper which is short and is positioned in the centre of the product. In order to save material, the product is folded around the release paper. The release paper is provided with a tab to make it easier to take hold of and to facilitate opening of the folded product when it is to be used. The arrangement is difficult to manufacture in a satisfactory manner because the folding of the product involves a number of process steps. It also limits the design possibilities of the product and the possibilities for varying the positioning of the means of attachment and the release strip. Moreover, the manufacturing cost is high with this type of process.

WO 99/37261 describes an incontinence pad with several adhesive areas. In order that the consumer is spared having to pull off several small release papers, a release paper extends over more than one adhesive area. The design is also stated to facilitate removal of the release paper, because there is an adhesive-free area under parts of the release paper. The disadvantage of this type of product is that it has a number of areas with means of attachment. It is involved for the consumer to have to take off several release papers and to have to fasten a number of adhesive areas in the briefs.

One of the major problems for the invention to solve is therefore to find a way which is easy from the point of view of the consumer of taking off a release paper so as then to be capable of fastening the product in the briefs. It is a great advantage if this solution is then moreover easy to produce.

### THE OBJECT AND MAJOR CHARACTERISTICS OF THE INVENTION

The object of the invention is to remedy the above mentioned problems and to design an absorbent product which is user-friendly.

According to the invention, an absorbent product of the type referred to in the introduction is provided, which product is characterized mainly in that the protective layer at least along a part of its outer periphery has an edge with a wave shape.

In this context, wave shape means all types of wave shapes, such as sine waves, sawtooth waves, square waves or the like. The wave-shaped edge can extend along only a part of the periphery of the protective layer and can in this connection be arranged essentially in the longitudinal direction of the product or essentially in the transverse direction. A protective layer usually has a generally elongate shape with two side edges extending essentially in the longitudinal direction and two end edges extending essentially in the According to a preferred embodiment, the entire edge of the protective layer is wave-shaped. Such an embodiment is especially advantageous because it makes it possible to take hold of the projecting portions formed by the waves anywhere along the edge of the protective layer.

The purpose of the edge waves on the protective layer is to create a multiplicity of tabs which can be taken hold of and project beyond the means of attachment and are therefore not fastened directly to it. The tabs should be flexible, so that they can be bent aside from the lower side of the absorbent product, in which way one or more tabs can easily be taken hold of when the protective layer is to be removed from the absorbent product.

The shape of the wave-shaped edge of the protective layer can of course be varied along the edge. For example, the waves can have different periodicity, different amplitude, or a combination thereof. It is also possible to combine different wave shapes with one another, for example the sine waves, sawtooth waves and square waves mentioned above. The main point is that the protective layer has at least one edge portion with a multiplicity of tabs which can be taken hold of.

The attachment can be in the form of, for example, adhesive, touch-and-close fasteners, snap fasteners or other means suitable for attachment in a pair of briefs. In a preferred embodiment of the invention, adhesive is used. It is common to all these means of attachment that they should be protected in a suitable manner before use. One way in which it is possible to protect them is by using what is known as a protective layer or release layer. The release layer is preferably siliconized, but other variants of release layer are of course also possible, for example waxed paper, stamped or release-agent-treated plastic film or textile strips.

In a preferred embodiment of the invention, the edges of the release layer have a sine-wave shape as mentioned above. This shape offers the user a large number of tabs which can be taken hold of and used for pulling the release paper off. It is an advantage if the adhesive on the product does not go all the way out to the edges of the product, so that a partly adhesive-free area is obtained under the release paper. One way of producing such a design is to apply the adhesive in strands or in another pattern. It is also possible to have the adhesive area stop a little inside the edges of the product. Another variant is to have the release paper project beyond the periphery of the product. In a further embodiment of the invention, the edges of the release layer have a sine-wave shape or the like in both the longitudinal direction and the transverse direction. According to another embodiment of the invention, only the edges in the transverse direction of the release layer have a sine-wave shape or the like.

An alternative embodiment of the edge of the release layer is that it can be sawtooth-shaped, or have a different type of wave shape.

### BRIEF DESCRIPTION OF DRAWINGS

The invention will be described in greater detail below with reference to the figures shown in the accompanying drawings.
- Figure 1: shows a sanitary towel according to the invention seen from below.
- Figure 2: shows a section along the line II-II through the sanitary towel in Figure 1.
- Figure 3: shows a sanitary towel according to an alternative embodiment of the invention seen from below.
- Figure 4: shows a sanitary towel according to an alternative embodiment of the invention seen from below.
- Figure 5: shows a sanitary towel according to an alternative embodiment of the invention seen from below.
- Figure 6: shows an incontinence pad according to an alternative embodiment of the invention seen from below.

### DESCRIPTION OF EMBODIMENTS

The invention relates to an absorbent product such as a sanitary towel, an incontinence pad, a panty liner or the like intended to be worn in a pair of briefs.

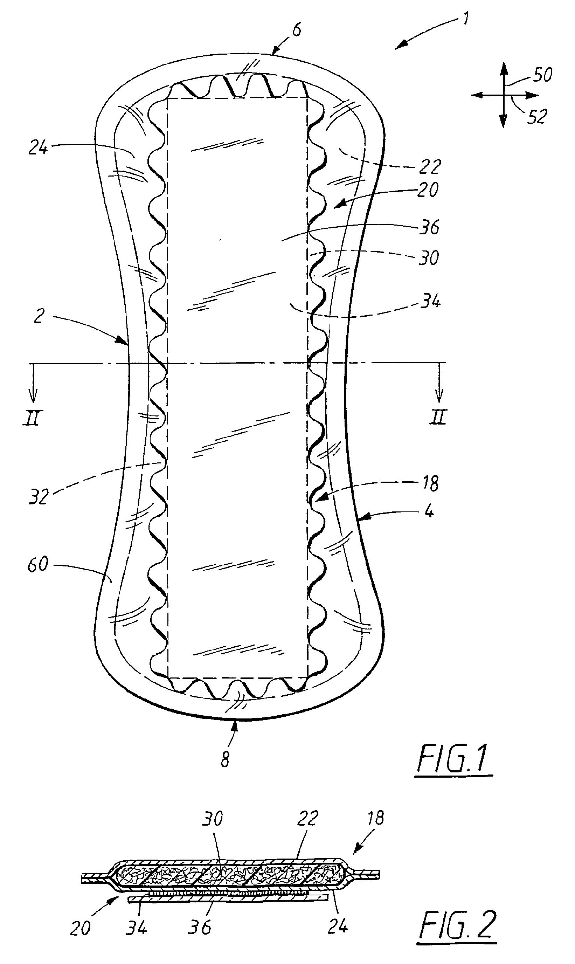

The embodiment shown in Figure 1 is in the form of a sanitary towel 1 which has an essentially elongate shape with a longitudinal direction 50 and a transverse direction 52 and also has two long sides 2, 4 and two short sides 6, 8. The sanitary towel 1 has an upper side 18, intended to face the wearer during use, and a lower side 20, intended to face away from the wearer during use.

The sanitary towel 1 comprises a liquid-permeable surface layer 22 arranged on the upper side 18 of the sanitary towel 1, that is to say that side of the sanitary towel 1 which is intended to face the wearer during use. A liquid-blocking backing layer 24 is arranged on the lower side 20 of the sanitary towel, that is to say the side which is intended to face away from the wearer during use. Arranged between the surface layer 22 and the liquid-blocking backing layer 24 is an absorbent body 30. The surface layer 22 and the backing layer 24 can, as shown in Figures 1 and 2, be joined together at a join 60 outside the periphery of the absorbent body 30.

The surface layer 22 can be made of any conventional material, for example non-woven fabric, net, perforated plastic film or a laminate made of a perforated plastic film and a non-woven fabric.

The absorbent body 30 is suitably made from one or more plies of cellulose pulp. The pulp can initially be in the form of rolls, bales or sheets which, during manufacture of the sanitary towel, are defibred and converted in fluffed form into a pulp mat, sometimes with what are known as superabsorbents being added, which are polymers with the capacity to absorb several times their own weight of water or bodily fluid. An alternative to this is to dry-form a pulp mat as described in WO 94/10956. Examples of other absorbent materials that can be used are different types of natural fibres such as cotton fibres, peat or the like. It is of course also possible to use absorbent synthetic fibres, or particles of a highly absorbent polymer material of the type which during absorption chemically binds great quantities of liquid as it forms a liquid-containing gel, or mixtures of natural fibres and synthetic fibres.

The absorbent body 30 can also include further components, such as shape-stabilizing means, liquid-spreading means, or binders such as, for example, thermoplastic fibres which have been heat-treated in order to hold short fibres and particles together in a coherent unit. It is also possible to use different types of absorbent foamed material in the absorbent body.

The liquid-blocking backing layer 24 consists of a liquid-impermeable material. Thin, liquidtight plastic films are suitable for the purpose, but it is also possible to use material which is initially liquid-permeable but has been provided with a coating of plastic, resin or other liquidtight material. In this way, leakage of liquid from the lower side of the absorbent product is prevented. The barrier layer 24 can therefore consist of any material which meets the criterion of liquid-impermeability and is also sufficiently flexible and skin-friendly for the purpose. Examples of materials which are suitable as barrier layers are plastic films, non-woven fabrics and laminates made of these. The plastic film can be made of, for example, polyethylene, polypropylene or polyester. Alternatively, the barrier layer can consist of a laminate made of a liquid-impermeable plastic layer facing the absorbent body, and a non-woven fabric facing the underwear of the wearer. Such a construction provides a leakproof barrier layer with a textile feel. The liquid-blocking backing layer 24 can also consist of a vapour-permeable material. Such a breathable backing layer 24 can be made of, for example, what is known as an SMS material (spunbond-meltblown-spunbond) or a breathable plastic film consisting of polyethylene. Such a plastic film is described in EP 283 200. In order to preserve the breathability even when the material has been applied to an absorbent product, the lower side 20 of the product should not be entirely covered by means of attachment 34.

An admission layer 32 can be arranged between the surface layer 22 and the absorbent body 30. The function of the admission layer 32 is to draw liquid into the sanitary towel and to transport it down to the absorbent body 30. The admission layer 32 can be made of a non-woven material of low density.

A means of attachment 34 is applied on the lower side 20 of the sanitary towel. The means of attachment preferably consists of adhesive but it can also be a mechanical attachment such as, for example, touch-and-close fasteners, snap fasteners, friction coatings, clip means or the like. The adhesive can be applied in one or more strands or in another pattern. Alternatively, the entire lower surface 20 of the towel 1 is coated with adhesive. It is also possible to use an attachment adhesive which is breathable and to apply it over the entire lower side 20 of the product so that it serves as a combined barrier layer and means of attachment.

A protective layer 36 is applied to the means of attachment 34. The protective layer 36 is preferably a siliconized paper, but other variants of protective layer 36 are of course also possible, for example waxed paper, stamped or release-agent-treated plastic film, textile strips to attach to touch-and-close fasteners etc. In the embodiment shown in Figure 1, the protective layer 36 has a sine-wave shape along both the long sides 2, 4 and the short sides 6,8.

Figure 2 shows a section along the line II-II through the sanitary towel 1 in Figure 1, but without an admission layer 32.

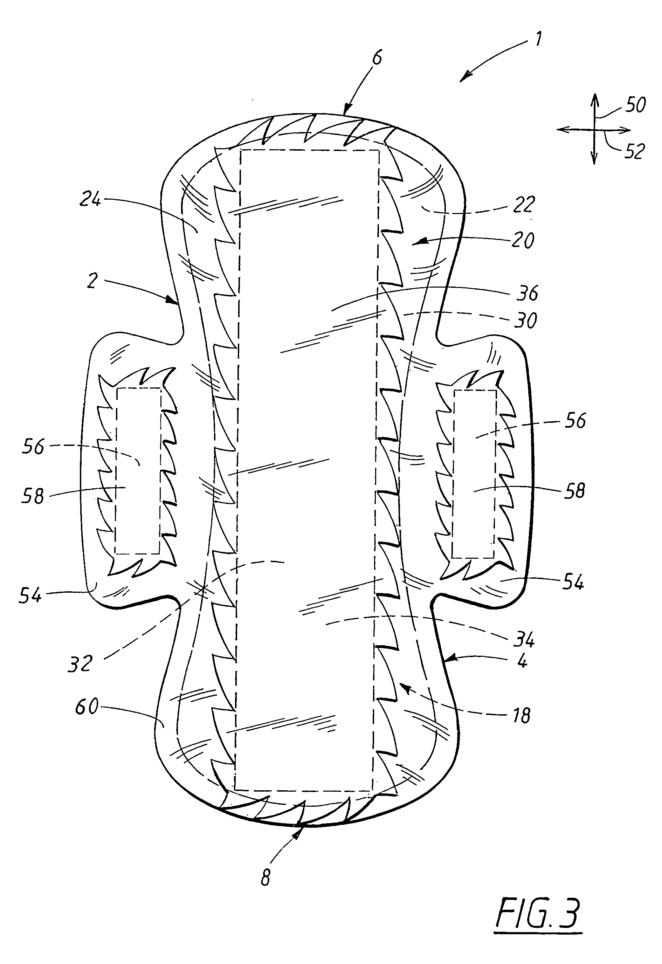

Figure 3 shows a sanitary towel 1 consisting of a surface layer 22, a backing layer 24 and an intermediate absorbent body 30. The sanitary towel is also provided with attachment flaps or what are known as wings 54. The attachment flaps 54 are fastened to the sanitary towel in a conventional manner, which may be, for example, that they constitute a continuation of the surface layer 22 and the backing layer 24. The attachment flaps 54 are provided with means of attachment 56 which can be designed in a corresponding manner to the means of attachment 34 of the sanitary towel. The means of attachment 56 of the wings 54 are also covered by protective layers 58. The means of attachment 34, 56 are covered by protective layers 36, 58 which have an edge with a sawtooth shape.

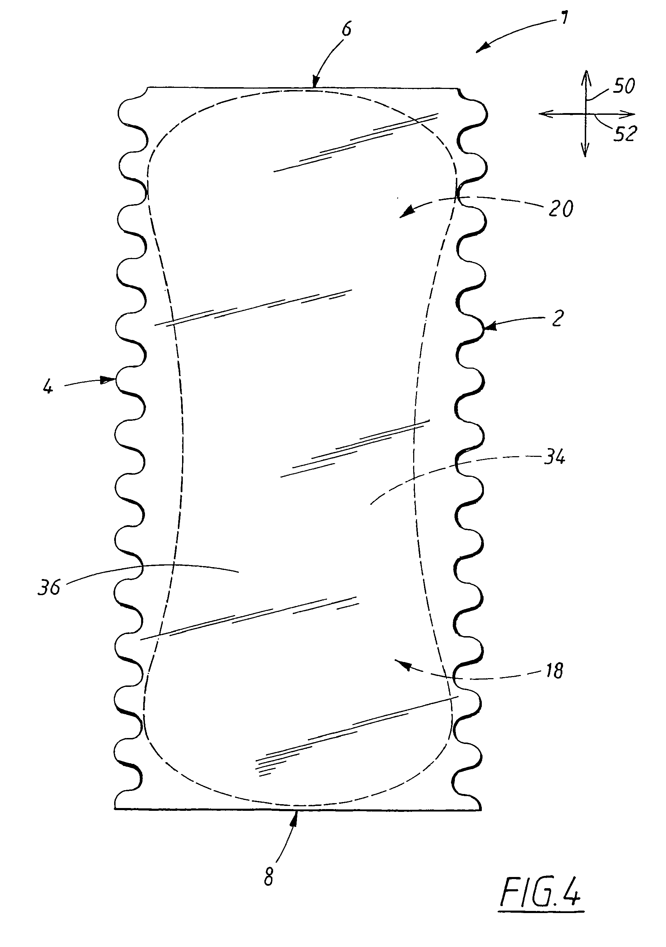

The embodiment shown in Figure 4 shows a sanitary towel 1 in which the means of attachment 34 consists of adhesive. The adhesive is applied over the entire lower side 20 of the product. The protective layer 36 in this embodiment has an outer contour which at least in part extends outside the periphery of the sanitary towel. The protective layer 36 has a sine-wave shape on both the long sides 2,4 and the short sides 6, 8.

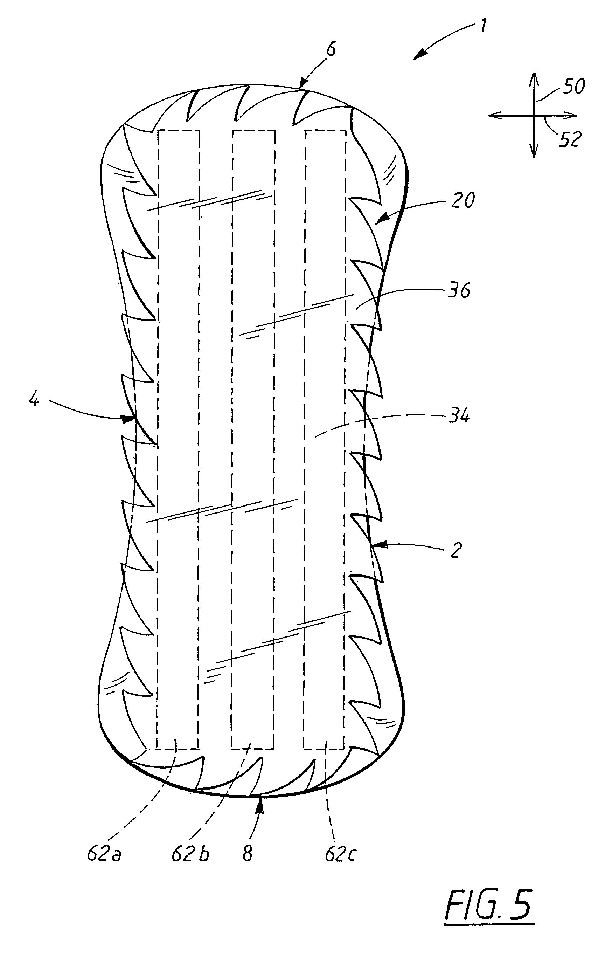

An alternative to the embodiment shown in Figure 4 is that the means of attachment 34 is applied in the form of three adhesive strands 62a, 62b, 62c, which do not go all the way out to the edge on the lower side 20 of the sanitary towel 1. This embodiment is shown in Figure 5.

Other shapes of means of attachment 34 are also possible, for example elliptical, circular or rectangular. It is also possible to apply these shapes in the form of one large area or alternatively in a number of smaller areas.

The release paper in Figure 5 has the same extent on the long sides 2, 4 and the short sides 6, 8 as the sanitary towel 1, apart from the edge of the protective layer 36 having a sawtooth shape.

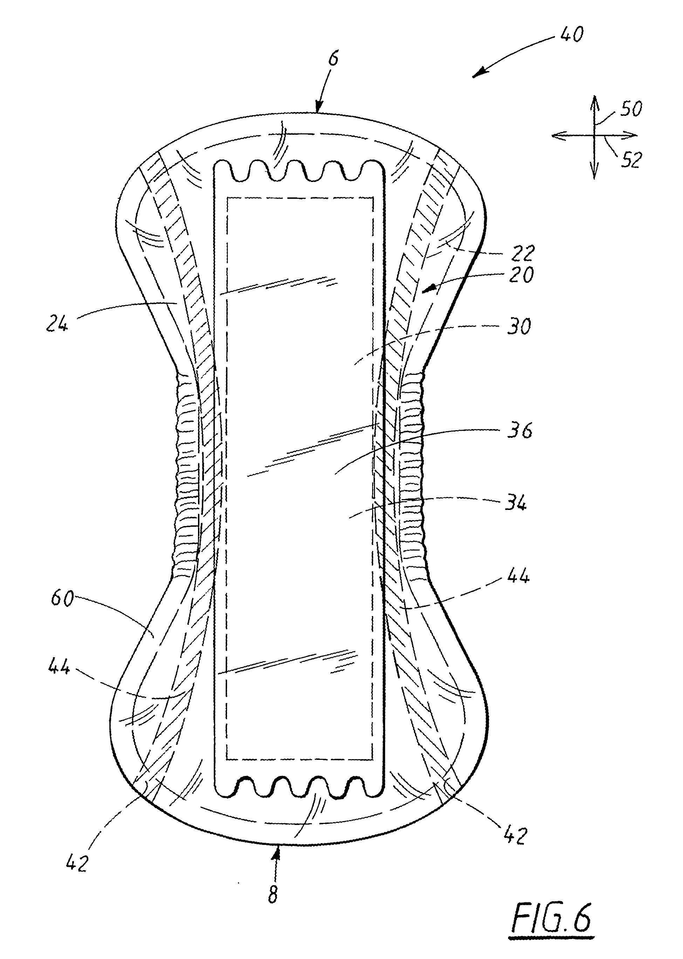

The product shown in Figure 6 is an hourglass-shaped incontinence pad 40. The surface layer 22 and the backing layer 24 are interconnected at a join 60 outside the periphery of the absorbent body 30. The covering layers 22, 24 can be interconnected in a number of different ways. Examples of connection methods are gluing, thermobonding, ultrasonic welding or the like.

The incontinence pad 40 has barriers 42 which are arranged on the liquid-permeable surface layer 22 and extend in the longitudinal direction of the incontinence pad 40.

The barriers 42 comprise elastic elements 44 which are connected to the barriers 42 in a tensioned state. When the tensioned elastic elements 44 are released, they contract together with the barriers 42 to which the elastic elements 44 are connected. The barriers 42 are then brought into a raised configuration so that they extend away from the surface layer 22. The elastic elements 44 can consist of, for example, one or more elastic threads, one or more elastic bands, strips of elastic plastic films, strips of elastic non-woven material, elastic foamed material or the like. The elastic elements 44 can be connected to the barriers 42 in a number of different ways. Examples of connection methods are gluing, welding, stitching or the like. Other types of barrier are of course also possible.

The lower side 20 has a means of attachment 34 which is covered by a protective layer 36 which has a sine-wave shape along the short sides 6, 8 of the incontinence pad 40.

The invention is not to be considered as being limited to the embodiments above. These are intended only to explain the invention. It is possible to combine characteristics from different embodiments with one another within the scope of the invention.

## Claims

1. Absorbent product such as a sanitary towel, an incontinence pad, a panty liner or the like, which has a longitudinal direction (50) and a transverse direction (52), the product comprising a surface layer (22) intended to face a wearer, a backing layer (24) intended to face away from the wearer, and an absorbent body (30) arranged between the surface layer (22) and the backing layer (24), a means of attachment (34) for application of the product, and a protective layer (36) for the means of attachment (34), **characterized in that** the protective layer (36) at least along a part of its outer periphery has an edge with a wave shape.

2. Absorbent product according to Claim 1, **characterized in that** the wave shape comprises a sine-wave shape.

3. Absorbent product according to Claim 1 or 2, **characterized in that** the wave shape comprises a sawtooth shape.

4. Absorbent product according to Claim 1, 2 or 3, **characterized in that** the wave shape comprises a square-wave shape.

5. Absorbent product according to any one of the preceding claims, **characterized in that** the edge of the protective layer (36) extends in the longitudinal direction (50) of the product.

6. Absorbent product according to any one of the preceding claims, **characterized in that** the edge of the protective layer (36) extends in the transverse direction (52) of the product.

7. Absorbent product according to any one of the receding claims, **characterized in that** the protective layer (36) has an edge with a wave
shape in both the longitudinal direction (50) and the transverse direction (52) of the product.

8. Absorbent product according to any one of the preceding claims, **characterized in that** the protective layer (36) comprises a waxed paper.

9. Absorbent product according to any one of the preceding claims, **characterized in that** the protective layer (36) comprises a siliconized paper.

10. Absorbent product according to any one of the preceding claims, **characterized in that** the protective layer (36) projects beyond the periphery of the product.

11. Absorbent product according to any one of the preceding claims, **characterized in that** all the edges of the protective layer (36) are wave-shaped.

12. Absorbent product according to any one of the preceding claims and also having attachment flaps (54), the attachment flaps (54) being provided with means of attachment (56), and a protective layer (58) being arranged over each means of attachment (56) on the attachment flaps (54), **characterized in that** the protective layer (58) on the attachment flaps (54) has an edge with a wave-shape.

## Patentansprüche

1. Absorptionsprodukt, wie zum Beispiel eine Binde, ein Inkontinenzpad, eine Slipeinlage oder dergleichen, das eine Längsrichtung (50) und eine Querrichtung (52) aufweist, wobei das Produkt eine Oberflächenlage (22), die dazu gedacht ist, einem Benutzer zugewandt zu sein, eine Stützlage (24), die dazu gedacht ist, von dem Benutzer weg gerichtet zu sein, und einen Absorptionskörper (30), der zwischen der Oberflächenlage (22) und der Stützlage (24) angeordnet ist, ein Anbringungsmittel (34) zum Anbringen des Produkts und eine Schutzlage (36) für das Anbringungsmittel (34) umfasst, **dadurch gekennzeichnet, dass** die Schutzlage (36) zumindest entlang einem Teil ihres äußeren Umfangs eine Kante mit einer Wellenform aufweist.

2. Absorptionsprodukt nach Anspruch 1, **dadurch gekennzeichnet, dass** die Wellenform eine Sinuswellenform umfasst.

3. Absorptionsprodukt nach Anspruch 1 oder 2, **dadurch gekennzeichnet, dass** die Wellenform eine Sägezahnform umfasst.

4. Absorptionsprodukt nach Anspruch 1, 2 oder 3, **dadurch gekennzeichnet, dass** die Wellenform eine Rechteckwellenform umfasst.

5. Absorptionsprodukt nach einem der vorhergehenden Ansprüche, **dadurch gekennzeichnet, dass** sich die Kante der Schutzlage (36) in der Längsrichtung (50) des Produkts erstreckt.

6. Absorptionsprodukt nach einem der vorhergehenden Ansprüche, **dadurch gekennzeichnet, dass** sich die Kante der Schutzlage (36) in der Querrichtung (52) des Produkts erstreckt.

7. Absorptionsprodukt nach einem der vorhergehenden Ansprüche, **dadurch gekennzeichnet, dass** die Schutzlage (36) eine Kante mit einer Wellenform sowohl in der Längsrichtung (50) als auch in der Querrichtung (52) des Produkts aufweist.

8. Absorptionsprodukt nach einem der vorhergehenden Ansprüche, **dadurch gekennzeichnet, dass** die Schutzlage (36) ein Wachspapier umfasst.

9. Absorptionsprodukt nach einem der vorhergehenden Ansprüche, **dadurch gekennzeichnet, dass** die Schutzlage (36) ein silikonisiertes Papier umfasst.

10. Absorptionsprodukt nach einem der vorhergehenden Ansprüche, **dadurch gekennzeichnet, dass** die Schutzlage (36) über den Umfang des Produkts hervorsteht.

11. Absorptionsprodukt nach einem der vorhergehenden Ansprüche, **dadurch gekennzeichnet, dass** alle Kanten der Schutzlage (36) wellenförmig sind.

12. Absorptionsprodukt nach einem der vorhergehenden Ansprüche und auch umfassend Anbringungsklappen (54), wobei die Anbringungsklappen (54) mit Anbringungsmitteln (56) versehen sind, und eine Schutzlage (58), die über jedem Anbringungsmittel (56) an den Anbringungsklappen (54) angeordnet ist, **dadurch gekennzeichnet, dass** die Schutzlage (58) an den Anbringungsklappen (54) eine Kante mit einer Wellenform aufweist.

## Revendications

1. Produit absorbant, comme une serviette hygiénique, une protection contre l'incontinence, un protège-slip ou élément similaire, ayant une direction longitudinale (50) et une direction transversale (52), le produit comprenant une couche de surface (22) destinée à faire face à un porteur, une couche de doublage (24) destinée à être éloignée du porteur, et un corps absorbant (30) placé entre la couche de surface (22) et la couche de doublage (24), un moyen de fixation (34) pour l'application du produit, et une couche protectrice (36) pour le moyen de fixation (34), **caractérisé en ce que** la couche protectrice (36), au moins le long d'une partie de sa périphérie extérieure, a un bord ayant une forme d'onde.

2. Produit absorbant selon la revendication 1, **caractérisé en ce que** la forme d'onde comprend une forme sinusoïdale.

3. Produit absorbant selon la revendication 1 ou 2, **caractérisé en ce que** la forme d'onde comprend une forme en dents de scie.

4. Produit absorbant selon la revendication 1, 2 ou 3, **caractérisé en ce que** la forme d'onde comprend une forme d'onde carrée.

5. Produit absorbant selon l'une quelconque des revendications précédentes, **caractérisé en ce que** le bord de la couche protectrice (36) s'étend dans la direction longitudinale (50) du produit.

6. Produit absorbant selon l'une quelconque des revendications précédentes, **caractérisé en ce que** le bord de la couche protectrice (36) s'étend dans la direction transversale (52) du produit.

7. Produit absorbant selon l'une quelconque des revendications précédentes, **caractérisé en ce que** la couche protectrice (36) a un bord ayant une forme d'onde à la fois dans la direction longitudinale (50) et dans la direction transversale (52) du produit.

8. Produit absorbant selon l'une quelconque des revendications précédentes, **caractérisé en ce que** la couche protectrice (36) comprend un papier ciré.

9. Produit absorbant selon l'une quelconque des revendications précédentes, **caractérisé en ce que** la couche protectrice (36) comprend un papier siliconé.

10. Produit absorbant selon l'une quelconque des revendications précédentes, **caractérisé en ce que** la couche protectrice (36) fait saillie au-delà de la périphérie du produit.

11. Produit absorbant selon l'une quelconque des revendications précédentes, **caractérisé en ce que** les bords de la couche protectrice (36) sont en forme d'onde.

12. Produit absorbant selon l'une quelconque des revendications précédentes, comprenant en outre des rabats de fixation (54), les rabats de fixation (54) étant pourvus d'un moyen de fixation (56), et une couche protectrice (58) étant placée sur chaque moyen de fixation (56) des rabats de fixation (54), **caractérisé en ce que** la couche protectrice (58) des rabats de fixation (54) a un bord en forme d'onde.
